# EUROPEAN PATENT APPLICATION

(11) **EP 4 186 494 A1**
(43) Date of publication of application: **31.05.2023**
(21) Application number: 21846082.2
(22) Date of filing: 21.07.2021
(51) Int. Cl.: A61K 9/14, A61K 31/7088, A61K 38/02, A61K 38/28, A61K 45/00, A61K 47/38, A61K 47/44, A61P 1/14, A61P 3/10, A61P 43/00

(54) **JANUS FINE PARTICLES AND METHOD FOR PRODUCING SAME**

(30) Priority: 22.07.2020 JP 2020124921
(71) Applicant: Matsumoto, Akihiro, Hyogo, 666-0257 (JP); Murakami Masahiro, Osakasayama-shi, Osaka 589-0023 (JP)
(72) Inventor: Matsumoto, Akihiro, Hyogo, 666-0257 (JP); Murakami Masahiro, Osakasayama-shi, Osaka 589-0023 (JP)
(74) Representative: Godemeyer Blum Lenze Patentanwälte Partnerschaft mbB - werkpatent
(86) International application number: PCT/JP2021/027260
(87) International publication number: WO 2022/019322

(57) **Abstract**

It is an object of the present invention to provide a Janus fine particle, in which only the hemisphere on the porous side can be efficiently impregnated with a drug aqueous solution/suspension at a high content, and a method for producing the same. According to the present invention, provided is a Janus fine particle having a particle diameter of 0.01 to 5000 µm, which is composed of a porous hemisphere portion and a non-porous hemisphere portion, wherein the wettability of the porous hemisphere portion to water is better than the wettability of the non-porous hemisphere portion to water.

## Description

### Technical Field

The present invention relates to a heteromorphic fine particle (Janus fine particle) composed of two or more types of different substances, and a method for producing the same.

### Background Art

Since a Janus fine particle (heteromorphic fine particle), in which one hemisphere is composed of a substance A and the other hemisphere is composed of a substance B that is different from the substance A can be used in various applications, the Janus fine particle has increasingly attracted attention in recent years (Non-Patent Documents 1 to 4). Several application examples of the Janus fine particle that have been reported so far may include a Janus fine particle used as an alternative to a surfactant (Non-Patent Document 5), a Janus fine particle used as a display elementary particle (Non-Patent Document 6), and a Janus fine particle used in magnetic therapy for cancer (Non-Patent Document 7). As methods for preparing such Janus fine particles, a method of using a multi-fluid nozzle and a method of utilizing phase separation between polymers have been reported (Non-Patent Documents 8 to 10).

### Prior Art Documents

### Non-Patent Documents

Non-Patent Document 1: Perec V et al., Science 315, 1393 (2007)
Non-Patent Document 2: Chen Q et al., Science 331, 199 (2011)
Non-Patent Document 3: Reynwaw BJ et al., Nature 447, 461 (2007)
Non-Patent Document 4: Sacanna S et al., Nature 464, 575 (2010)
Non-Patent Document 5: Ruhland T et al., Langmuir 27, 9807 (2011)
Non-Patent Document 6: Yin SN et al., Adv. Matter. 23, 2915 (2011)
Non-Patent Document 7: Hu SH et al., J. Am. Chem. Soc. 132, 7234 (2010)
Non-Patent Document 8: Takasi Nisisako et al., Adv. Mater. 2006, 18, 1152-1156
Non-Patent Document 9: Zhihong Nie et al., J. Am. Chem. SOC. 2006, 128, 9408-9412
Non-Patent Document 10: Chariya Kaewsaneha et al., ACS Appl. Mater. Interfaces, 2013, 5 (6), 1857-1869

### Summary of Invention

### Object to be Solved by the Invention

It is an object of the present invention to provide a Janus fine particle, in which only the hemisphere on the porous side can be efficiently impregnated with a drug aqueous solution/suspension at a high content, and a method for producing the same.

### Means for Solving the Object

As a result of intensive studies conducted directed towards achieving the aforementioned object, the present inventors have found that, by creating a Janus fine particle that is composed of a porous hemisphere portion and a non-porous hemisphere portion, and by adopting the configuration of the Janus fine particle by which the wettability of the porous hemisphere portion is good and the wettability of the non-porous hemisphere portion is poor, the created Janus fine particle, in which only the hemisphere on the porous side can be efficiently impregnated with a drug aqueous solution/suspension at a high content, can be obtained, thereby completing the present invention.

Specifically, according to the present invention, the following invention is provided.
<1> A Janus fine particle having a particle diameter of 0.01 to 5000 µm, which is composed of a porous hemisphere portion and a non-porous hemisphere portion, wherein the wettability of the porous hemisphere portion to water is better than the wettability of the non-porous hemisphere portion to water.
<2> The Janus fine particle according to <1>, wherein at least the non-porous hemisphere portion of the Janus fine particle further comprises a wax.
<3> The Janus fine particle according to <1> or <2>, wherein the porous hemisphere portion at least comprises a polymer that swells due to water absorption.
<4> The Janus fine particle according to any one of <1> to <3>, wherein the porous hemisphere portion at least comprises an enteric polymer.
<5> The Janus fine particle according to any one of <1> to <4>, wherein the porous hemisphere portion at least comprises hypromellose phthalate or hydroxymethyl cellulose acetate succinate.
<6> The Janus fine particle according to any one of <1> to <5>, wherein the porous hemisphere portion comprises a drug.
<7> The Janus fine particle according to <6>, wherein the drug is a peptide, a protein, a nucleic acid, or a nucleic acid derivative.
<8> The Janus fine particle according to any one of <1> to <5>, wherein the porous hemisphere portion comprises an absorption enhancer, a protease inhibitor, a mucoadhesive polymer, or a sol-gel agent.
<9> A method for producing the Janus fine particle any one of <1> to <7>, comprising the following steps (i) and (ii):
   (i) a step of emulsifying a solution prepared by dissolving one or more types of substances with good wettability and one or more types of substances with poor wettability in a common solvent, into a surfactant-containing aqueous solution; and
   (ii) a step of drying the obtained emulsion to remove the solvent.
<10> The method according to <8>, wherein the volume of the surfactant-containing aqueous solution is 0.5 to 10 times higher than the volume of the solution prepared by dissolving one or more types of substances with good wettability and one or more types of substances with poor wettability in a common solvent.
<11> The method according to <8> or <9>, wherein, in the step (ii), the emulsion is dried for 30 minutes or more to remove the solvent.
<12> The method according to any one of <8> to <10>, which further comprises, after the step (ii),
   (iii) a step of impregnating the porous hemisphere portion with an aqueous solution or suspension of a drug.
<13> The method according to any one of <8> to <11>, wherein the surfactant is a nonionic surfactant.
<14> The method according to any one of <8> to <12>, wherein the surfactant is polyvinyl alcohol.

### Advantageous Effects of Invention

According to the Janus fine particle of the present invention and the method for producing the same, a preparation for promoting efficient absorption of a drug by a biological membrane can be provided.

### Brief Description of Drawings

[Fig. 1] Fig. 1 shows an overview of the system of the present invention.
[Fig. 2] Fig. 2 shows Janus fine particles in Example 1.
[Fig. 3] Fig. 3 shows Janus fine particles in Example 1.
[Fig. 4] Fig. 4 shows Janus fine particles in Example 1.
[Fig. 5] Fig. 5 shows Janus fine particles in Comparative Example 1.
[Fig. 6] Fig. 6 shows Janus fine particles in Example 2.
[Fig. 7] Fig. 7 shows Janus fine particles in Example 3.
[Fig. 8] Fig. 8 shows Janus fine particles in Example 4.
[Fig. 9] Fig. 9 shows Janus fine particles in Example 5.
[Fig. 10] Fig. 10 shows Janus fine particles in Example 6.
[Fig. 11] Fig. 11 shows Janus fine particles in Example 7.
[Fig. 12] Fig. 12 shows Janus fine particles in Example 8.
[Fig. 13] Fig. 13 shows transitions of plasma glucose levels after administration of individual preparations.

### Embodiments of Carrying out the Invention

Hereinafter, the present invention will be described in more detail.

The Janus fine particle of the present invention is a Janus fine particle having a particle diameter of 0.01 to 5000 µm, which is composed of a porous hemisphere portion and a non-porous hemisphere portion. In the Janus fine particle of the present invention, the wettability of the porous hemisphere portion to water is better than the wettability of the non-porous hemisphere portion to water.

Promotion of the absorption of a drug by a biological membrane has been studied from a long time ago, and the use of an absorption enhancer, coadministration of a drug with an enzyme inhibitor, use of a mucoadhesive polymer, and the like have been proposed. The use of fine particles is also one of the proposed technologies. It has been considered that a drug is released from a fine particle near the absorption surface of a biological membrane, so that the enzymatic degradation of the drug can be suppressed and the amount of the drug absorbed by the membrane can be increased. However, there is no selectivity regarding the direction of the drug released from the fine particle. That is, some drugs are released towards the absorption surface, but other drugs are released toward the opposite side of the absorption surface. In addition, the thus released drugs are at a risk of degradation until they reach the absorption surface. Takada et al. have proposed a hemisphere preparation, in which the release of a drug from the spherical surface is suppressed and the release of the drug is limited to the release from the flat surface. While the flat surface portion as a drug-releasing surface is adhered to the absorption surface of a biological membrane, the spherical surface suppresses drug release to the opposite side of the biological membrane and at the same time, the spherical surface acts as a barrier to enzymes, so that absorption of the drug by the biological membrane is efficiently carried out. However, it is technically difficult to produce a fine hemisphere preparation.

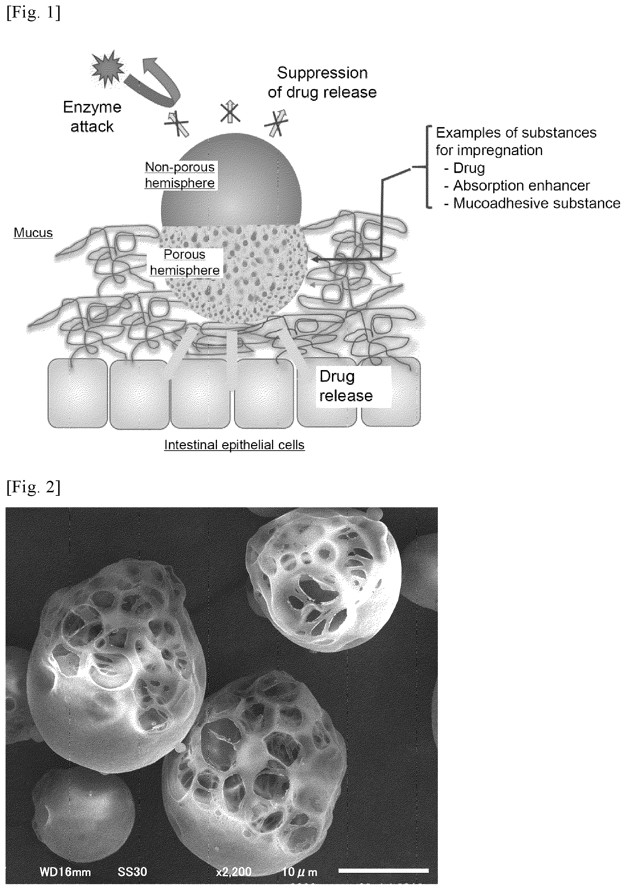

In the present invention, by utilizing the characteristics of a Janus fine particle, one hemisphere is configured to suppress drug release to the opposite side of a biological membrane, and at the same time, to act as a barrier to enzymes, whereas the other hemisphere is configured to be melted at a temperature close to body temperature and to be absorbed by the biological membrane when it is contacted with the biological membrane, so that the system shown in Fig. 1 has been conceived. At that time, the drug needs to be distributed in a high concentration to the side of the mucosa. As a method of incorporating a drug into a fine particle, there is conceived a method which comprises dissolving a fine particle base agent in a solvent to produce an oil phase, emulsifying the oil phase in a small amount of water phase, gradually performing liquid drying, and then adding a surfactant, an insoluble solid or water droplets into the oil phase, so as to form a solid in oil (S/O) emulsion or a water in oil (W/O) emulsion. According to this method, however, it is difficult to incorporate 30% or more of the drug into the fine particle. Hence, as a result of creative studies, the present inventors have succeeded in producing a Janus fine particle consisting of a hemisphere with good wettability and a substance with poor wettability, wherein the hemisphere consisting of a substance with good wettability is processed to be a porous hemisphere. Moreover, the present inventors have conceived that a wax such as a hard fat is added to the hemisphere with poor wettability to further deteriorate the wettability of the hemisphere, and then, a drug-containing solution is added to the Janus fine particle, so that the porous hemisphere with good wettability can be efficiently impregnated with the drug-containing solution. Furthermore, such deterioration of the wettability of the non-porous hemisphere is effective also upon production of the fine particle. That is to say, by deteriorating the wettability of the non-porous hemisphere, an aqueous solution used in the production of the fine particle (i.e. a water phase of an emulsion, and an aqueous solution for liquid drying) hardly infiltrates into the non-porous hemisphere, and as a result, pore formation is inhibited in the non-porous hemisphere.

Wettability is a phenomenon in which the interface that is a boundary between a solid and a gas is replaced with the interface between a solid and a liquid. When a liquid is added dropwise onto the surface of a solid, and when the liquid is stopped and is contacted with the surface of the solid at an angle θ, the angle θ is referred to as a "contact angle." The contact angle has been utilized as a value for evaluating wettability (edited by S. Noguchi, *Seizai-gaku*/*Butsuri Yakuzai-gaku Tsuron* (Outline of Galenical Pharmacy/Physical Pharmacy), Kyoto Hirokawa Shoten, pp. 18). As the contact angle increases, wettability becomes poor. In order to allow the porous hemisphere to be efficiently impregnated with a drug-containing solution, the contact angle of the solution and the non-porous hemisphere composition may be set to be greater than the contact angle of the solution and the porous hemisphere composition. Besides, simply, the contact angle of water and each hemisphere composition may also be used. A difference between the contact angle of the water and the porous hemisphere composition and the contact angle of water and the non-porous hemisphere composition may be 5° or more, preferably 10° or more, more preferably 20° or more, and further preferably 30° or more.

The method of measuring the contact angle is not particularly limited, and common methods such as a dropping method, a capillary method, or a heat of wetting measurement method may be applied. For simple evaluation of the contact angle, a method, which comprises preparing a film with the composition of each hemisphere, adding the solution dropwise onto the film, and then obtaining the contact angle according to a dropping method, may be applied.

The particle diameter of a Janus fine particle may be 0.01 to 5000 µm, and it is preferably 0.05 to 500 µm, and more preferably 0.1 to 100 µm.

The porous hemisphere portion preferably comprises a polymer that swells due to water absorption, for example, an enteric polymer.

Examples of the material for the porous hemisphere portion may include hypromellose phthalate and hydroxymethyl cellulose acetate succinate, but are not particularly limited thereto.

Examples of the material for the non-porous hemisphere portion may include polylactic acid, polyglycolic acid, a lactic acid-glycolic acid co-polymer, oligolectic acid, polyacrylate, polymethacrylate, an acrylate-methacrylate copolymer, polycaprolactone, polyvinyl pyrrolidone (PVP), and cellulosic polymers (e.g. ethyl cellulose, hydroxypropylmethyl cellulose (HPMC), hydroxypropyl cellulose (HPC), cellulose acetate phthalate, and hydroxypropylmethyl cellulose phthalate). Examples of the polyacrylate, the polymethacrylate and the acrylate-methacrylate copolymer, which can be used herein, may include an acrylate-ammonium methacrylate copolymer (Eudragit (registered trademark) RL100 or RS100, or Eudragit (registered trademark) RL30D or RS30D), an ethyl acrylate-methyl methacrylate copolymer (Eudragit (registered trademark) NE30D), a methacrylic acid copolymer (Eudragit (registered trademark) L100-55, Eudragit (registered trademark) L30D, Eudragit (registered trademark ) E100, or Eudragit (registered trademark) EPO), a starch polymer, and chitosan.

The non-porous hemisphere portion is preferably composed of ethyl cellulose, but is not particularly limited thereto.

It is preferable that the non-porous hemisphere portion further comprises a wax.

The type of such a wax is not particularly limited, and specific examples may include: saturated fatty acids containing 14 to 24 carbon atoms (e.g. myristic acid, palmitic acid, stearic acid, behenic acid, etc.) or salts thereof (e.g. a sodium salt and a potassium salt); higher alcohols containing 16 to 24 carbon atoms (e.g. cetyl alcohol, stearyl alcohol, etc.); monoglycerides, diglycerides or triglycerides of saturated and/or unsaturated fatty acids containing 8 to 24 carbon atoms; oils and fats (e.g. hydrogenated oils, such as castor oil, cottonseed oil, olive oil, soybean oil, rapeseed oil, or beef tallow); waxes (e.g. beeswax, carnauba wax, sperm oil, etc.); hydrocarbons (e.g. paraffin, microcrystalline wax etc.); cholesterol; glycolipids (e.g. sphingolipids, ceramides, etc.); phospholipids (e.g. phosphatidylcholine, phosphatidylethanolamine, phosphatidylserine, hydrogenated lecithin, etc. ); and hydrophobic vitamins (e.g. vitamin E, vitamin A, vitamin D, vitamin K, etc.). As a lipid, a fatty acid triglyceride (tri-O-acylglycerin) that is an ester of fatty acid and glycerin is particularly preferable. Such lipids can be used alone or by appropriately being combined and mixed with one another. Furthermore, low melting point lipids such as C6-C12 lower lipids (e.g. fatty acids, such as capric acid or lauric acid, or salts thereof, esters, alcohols, glycolipids, phospholipids, etc.) or vegetable oils (e.g. soybean oil, olive oil, castor oil, rapeseed oil, etc.) are added to the aforementioned lipids, so that the melting point, the consistency, or the interfacial tension of the oil and fat phase can be appropriately adjusted.

Preferred examples of the wax that can be used herein may include GELUCIRE and Suppocire AM. Suppocire AM PASILLES is a glyceride base containing saturated C8-C18 triglyceride fatty acid, having a melting point of 35.0°C to 36.5°C and a hydroxyl value of 5.

In the present invention, the porous hemisphere portion contains a drug.

Examples of the drug may include a peptide, a protein, a nucleic acid (DNA, a DNA decoy, a DNA plasmid, an aptamer, RNA, siRNA, tRNA, miRNA, a heteroduplex nucleic acid of DNA and RNA, etc.), nucleic acid derivatives (including chimeric compounds comprising DNA, RNA and a derivative thereof), and a peptide nucleic acid.

Specific examples of such a peptide, a protein, a nucleic acid or a nucleic acid derivative, which can be used as a drug herein, may include: proteins such as a growth hormone release factor, a growth factor, an epidermal growth factor (EGF), a nerve growth factor (NGF), TGF, PDGF, an insulin-like growth factor (IGF), a fibroblast growth factor (aFGF, bFGF, etc.), somatostatin, calcitonin, insulin, vasopressin, interferon, IL-2, urokinase, serratiopeptidase, superoxide dismutase (SOD), a thyrotrophin-releasing hormone (TRH), a luteinizing hormone release factor (LH-RH), a corticotropin-releasing hormone (CRF), a growth hormone releasing hormone (GHRH), oxytocin, erythropoietin (EPO) and a colony stimulating factor (CSF); peptides obtained from portions of such proteins; and peptide vaccines obtained from specific antigens. In addition, nucleic acids encoding these proteins or peptides and nucleic acid derivatives thereof can also be used.

The nucleic acid derivative means a molecule obtained by modifying ribonucleotide, deoxyribonucleotide, RNA or DNA, and the nucleic acid derivative may be either a naturally occurring molecule or a non-natural molecule.

An example of the nucleic acid derivative may be a molecule formed by adding another chemical substance to a nucleic acid. Specific examples of such a nucleic acid derivative may include a 5'-polyamine-added derivative, a cholesteroladded derivative, a steroid-added derivative, a bile acid-added derivative, a vitaminadded derivative, a Cy5-added derivative, a Cy3-added derivative, a 6-FAM-added derivative, and a biotin-added derivative.

Other examples of the nucleic acid derivative may include sugar modifications, such as oligonucleotide derivatives substituted with 2'-O-propyl ribose, 2'-methoxyethoxy ribose, 2'-O-methyl ribose, 2'-O-methoxyethyl ribose, 2'-O-[2- (guanidium)ethyl] ribose, or 2'-O-fluororibose. Moreover, examples of the nucleic acid derivatives may also include phosphate group modifications, such as an oligonucleotide derivative in which a phosphoric acid diester bond in the oligonucleotide has been converted to a phosphorothioate bond, and an oligonucleotide derivative in which a phosphoric acid diester bond in the oligonucleotide has been converted to an N3'-P5' phosphoramidate bond.

The porous hemisphere portion can also be impregnated with an absorption enhancer, a protease inhibitor, a mucoadhesive polymer, a sol-gel agent, etc., as well as with the drug.

The Janus fine particle of the present invention can be produced by the following steps:
(i) a step of emulsifying a solution prepared by dissolving one or more types of substances with good wettability and one or more types of substances with poor wettability in a common solvent, into a surfactant-containing aqueous solution; and
(ii) a step of drying the obtained emulsion to remove the solvent.

After completion of the above-described step (ii), the production of the present Janus fine particle may further comprise:
(iii) a step of impregnating the porous hemisphere portion with an aqueous solution or suspension of a drug.

The "aqueous solution or suspension of a drug" or the "solution prepared by dissolving one or more types of substances with good wettability and one or more types of substances with poor wettability in a common solvent" may comprise a substance having surfactant action, for example, an amphipathic substance such as a surfactant. Thereby, impregnation of the porous hemisphere with a drug solution can be promoted. In addition, the "aqueous solution or suspension of a drug" may comprise substances that impart to the fine particle, functionalities towards living bodies, such as, for example, an absorption enhancer and a mucoadhesive substance.

In the step (i), a solution prepared by dissolving one or more types of substances with good wettability and one or more types of substances with poor wettability in a common solvent is used. The common solvent is not particularly limited, as long as it is a solvent capable of dissolving therein both of the used one or more types of substances with good wettability and the used one or more types of substances with poor wettability. Examples of the common solvent that can be used herein may include methylene chloride, ethyl acetate, hexane, cyclohexane, ethanol, methanol, propanol, acetone, THF, DMF, DMSO, acetic acid, and a mixed solvent of these solvents.

The liquid that emulsifies the solution prepared by dissolving one or more types of substances with good wettability and one or more types of substances with poor wettability in a common solvent is not particularly limited, as long as it is a liquid that can be phase-separated from the solution prepared by dissolving one or more types of substances with good wettability and one or more types of substances with poor wettability in a common solvent. Examples of the liquid that can be used herein may include water, glycerin, silicon oil, castor oil, soybean oil, and olive oil.

The surfactant used in the present invention is not particularly limited, as long as it can emulsify the solution comprising one or more types of substances with good wettability and one or more types of substances with poor wettability. The surfactant may be any of an anionic surfactant, a cationic surfactant, an amphoteric surfactant, and a nonionic surfactant. As such a surfactant, a single type of surfactant may be used, or two or more types of different surfactants may also be used in combination.

Examples of the anionic surfactant may include soap (sodium fatty acid) RCOO⁻Na⁺, monoalkyl sulfate ROSO₃⁻M⁺, alkyl polyoxyethylene sulfate RO(CH₂CH₂O)ₘSO₃⁻M⁺, alkylbenzene sulfonate RR'CH₂CHC₆H₄SO₃⁻M⁺, and monoalkyl phosphate ROPO(OH)O⁻M⁺.

Examples of the cationic surfactants may include alkyl trimethyl ammonium salt RN⁺(CH₃)₃X⁻, dialkyl dimethyl ammonium salt RR'N⁺(CH₃)₂X⁻, and alkyl benzyl dimethyl-ammonium salt RN⁺(CH₂Ph)(CH₃)₂X⁻.

Examples of the amphoteric surfactant may include alkyl dimethylamine oxide R(CH₃)₂NO and alkyl carboxybetaine R(CH₃)₂N⁺CH₂COO⁻.

Examples of the nonionic surfactant may include polyoxyethylene alkyl ether RO(CH₂CH₂O)ₘH, fatty acid sorbitol ester, alkyl polyglucoside, fatty acid diethanolamide RCON(CH₂CH₂OH)₂, alkyl monoglyceryl ether ROCH₂CH(OH)CH₂OH, and polyvinyl alcohol.

The surfactant is particularly preferably a nonionic surfactant, and further particularly preferably polyvinyl alcohol.

The volume of the surfactant-containing aqueous solution in the above-described step (i) is preferably 0.5 to 10 times, and more preferably 1 to 10 times higher than the solution prepared by dissolving one or more types of substances with good wettability and one or more types of substances with poor wettability in a common solvent.

In the step (ii), the time required for removing the common solvent is not particularly limited, as long as desired Janus fine particles can be produced. It is preferable that the emulsion is dried for 30 minutes or more to remove the solvent. The time for removing the common solvent is more preferably 30 minutes to 6 hours, and further preferably 1 to 4 hours.

In the present invention, the "solution prepared by dissolving one or more types of substances with good wettability and one or more types of substances with poor wettability in a common solvent," the "surfactant aqueous solution," or the "aqueous solution or suspension of a drug" may comprise, as additives, a mucolytic agent (e.g. L-cysteine, N-acetylcysteine, etc.), a permeation enhancer or an absorption enhancer (e.g. medium chain fatty acid, long chain unsaturated fatty acid, their monoglycerides, polyethylene glycol esters, or mixtures of these substances, such as LABRASOL, bile salts, chelating agents, glycolipids including alkyl saccharide as a typical example, Azone (registered trademark), TRANSCUTOL (registered trademark), cyclodextrin, tight junction modifiers such as a Claudin binder and a derivative thereof, etc.), buffers (e.g. a phosphate buffer, a citrate buffer, a carbonate buffer, etc.), degrading enzyme inhibitors (e.g. aprotinin, bacitracin, camostat, citric acid, tartaric acid, sodium edetate, sodium glycocholate, SUPERase-In (registered trademark), RNasin (registered trademark), etc.), specific gravity regulators (e.g. glycerin, sucrose, glucose, amino acid, porous silica, etc.), antioxidants (e.g. ascorbic acid, hydroxybutyl anisole, tocopherol, ascorbic acid, CoQ10, fullerene, a fullerene derivative, etc.), and light-shielding and/or light-absorbing agents (titanium oxide, oxybenzone, octocrylene, etc.).

The present invention will be more specifically described in the following examples. However, these examples are not intended to limit the scope of the present invention.

### Examples

### < Example 1 >

60 mg of Hypromellose phthalate (Shin-Etsu Chemical Co., Ltd., HP55), 200 mg of ethyl cellulose (Colorcon, ETHOCEL STD7), 120 mg of GELUCIRE 39/01 (GATTEFOSSE), and 0.1 mg of Oil Red serving as a pigment were dissolved in a mixed solution consisting of 2 mL of methylene chloride and 0.5 mL of ethanol. The thus obtained solution was emulsified into 5 mL of 0.5% polyvinyl alcohol (KURARAY CO., LTD., KURARAY POVAL 220C) - 0.1 M hydrochloric acid solution, at 8000 rpm for 3 minutes, using a homogenizer (IKA, Ultratalax T18). After completion of the emulsification, 20 mL of purified water was added to the reaction mixture, and the thus obtained mixture was then stirred using a stirrer for 3 hours, so that the solvent was distilled away. Aggregates were removed using a wire mesh sieve having a sieve mesh size of 149 µm, and fine particles were then recovered using a wire mesh sieve having a sieve mesh size of 20 µm. The fine particles were washed with purified water, and were then dispersed in a small amount of purified water, followed by freeze-drying for 1 day. The obtained fine particles were observed under a scanning electron microscope. As a result, it was found that the observed fine particle was a fine particle having a particle diameter of about 20 µm, which was a sphere consisting of one porous hemisphere and the other hemisphere that had no pores (Fig. 2).

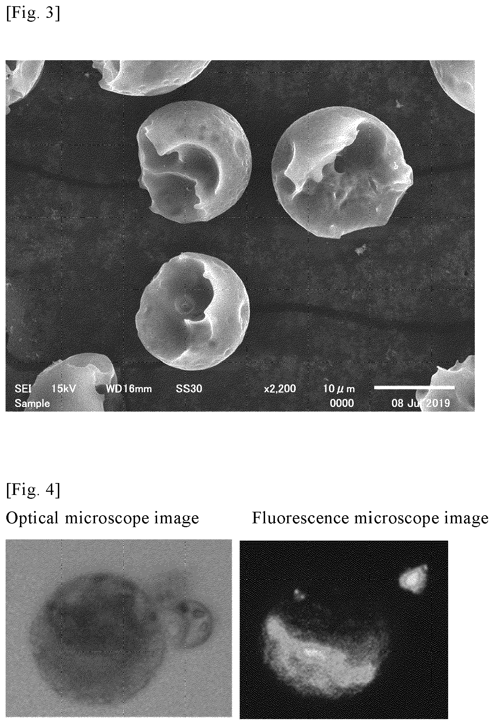

These fine particles were dispersed in a 0.1 M phosphate buffer (pH 8.0), were then washed with water, and were then freeze-dried. The obtained fine particles were observed under a scanning electron microscope. As a result, porous hemispheres were lysed, and hemispheres were thereby obtained (Fig. 3). From these results, it is considered that the porous hemisphere is constituted with HP55 that is soluble in alkaline.

The fine particles shown in Fig. 2 were impregnated with an aqueous soltuion of fluorescent-labeled dextran (FD4) having a molecular weight of 4000, so that the content of FD4 became 50%. The fluorescence microscope image of the obtained fine particle is shown in Fig. 4. It could be confirmed that FD4 was distributed only in the hemisphere. It is considered that the hemisphere, in which FD4 was distributed, was a porous hemisphere constituted with HP55 with good wettability. On the other hand, FD4 was not distributed in the other hemisphere. This is considered because the wettability of the other hemisphere to water became poor as a result of the mixing of hard fat such as Suppocire AM. Moreover, it was demonstrated that since it was a porous hemisphere, a high content of drug could be included in the porous hemisphere according to the impregnation method.

### < Comparative Example 1 >

### (Preparation of ethyl cellulose porous microsphere)

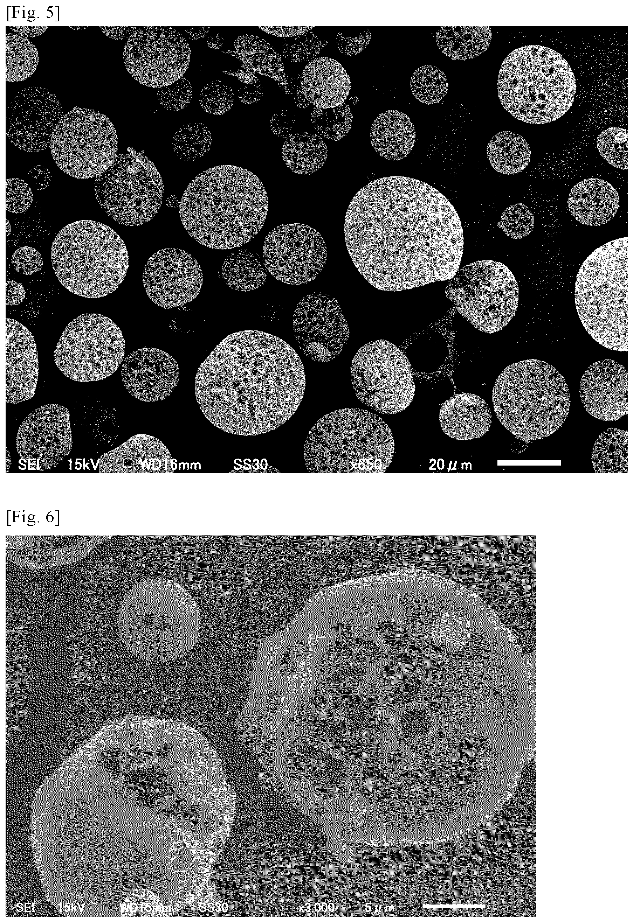

2 g of Ethyl cellulose (Nisshin Kasei Co., Ltd., ETHOCEL 7 cps) was dissolved in 16 g of acetone (solution A). Thereafter, 1 g of 5% polyvinyl alcohol (KURARAY CO., LTD., KURARAY CO., LTD., Poval 220C) was mixed with 7 g of glycerin, and the obtained mixture was then added to the solution A. Using a homogenizer (IKA, Ultratalax T18), the mixed solution was emulsified at 12000 rpm for 1 minute (solution B). Thereafter, 45 g of glycerin was mixed with 5 g of a 5% polyvinyl alcohol aqueous solution, and while the mixed solution was stirred with a propeller agitator (HEIDON, Three-One-Motor) (600 rpm), the solution B was added into the solution, followed by emulsifying for 1 minute. Immediately after completion of the emulsification, while the emulsion was stirred with a propeller agitator (HEIDON, Three-One-Motor) (400 rpm), the emulsion was added into 500 mL of purified water under stirring, so that porous microspheres (hereinafter referred to as "PMS") were precipitated. Aggregates were removed using a wire mesh sieve having a sieve mesh size of 149 µm, and the fine particles were then recovered using a wire mesh sieve having a sieve mesh size of 20 µm. The fine particles were washed with purified water, and were then dispersed in a small amount of purified water, followed by freeze-drying for 1 day. The obtained fine particles were observed under a scanning electron microscope. As a result, fine particles, which were entirely porous fine particles and had a particle diameter of about 20 µm, were obtained (Fig. 5).

### < Example 2 >

60 mg of Hypromellose phthalate (Shin-Etsu Chemical Co., Ltd., HP55), 200 mg of ethyl cellulose (Colorcon, ETHOCEL STD7), and 120 mg of Suppocire AM (GATTEFOSSE) were dissolved in a mixed solution consisting of 2 mL of methylene chloride and 0.5 mL of ethanol. The thus obtained solution was emulsified into 5 mL of 0.5% polyvinyl alcohol (KURARAY CO., LTD., KURARAY POVAL 220C) - 0.1 M hydrochloric acid solution, at 8000 rpm for 3 minutes, using a homogenizer (IKA, Ultratalax T18). After completion of the emulsification, 20 mL of purified water was added to the reaction mixture, and the thus obtained mixture was then stirred using a stirrer for 3 hours, so that the solvent was distilled away. Aggregates were removed using a wire mesh sieve having a sieve mesh size of 149 µm, and fine particles were then recovered using a wire mesh sieve having a sieve mesh size of 20 µm. The fine particles were washed with purified water, and were then dispersed in a small amount of purified water, followed by freeze-drying for 1 day. The obtained fine particles were observed under a scanning electron microscope (Fig. 6).

### < Example 3 >

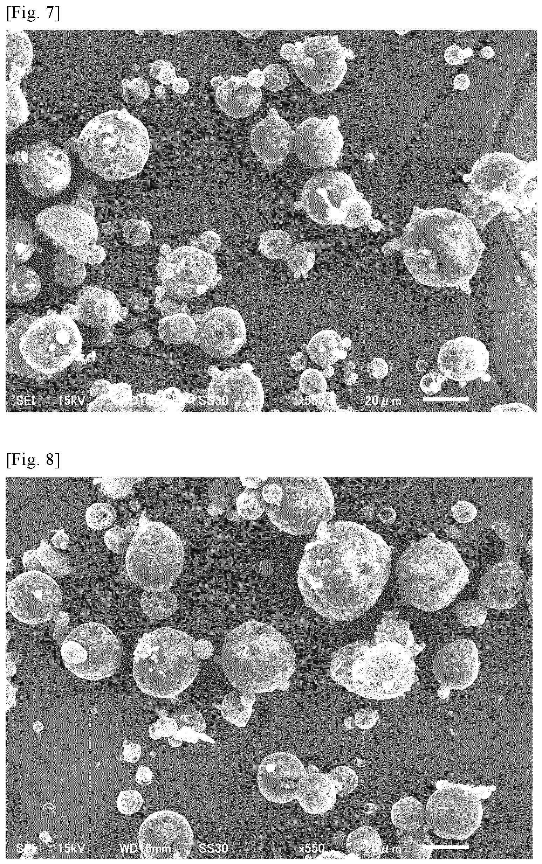

80 mg of Hypromellose phthalate (Shin-Etsu Chemical Co., Ltd., HP55), 200 mg of ethyl cellulose (Colorcon, ETHOCEL STD7), and 120 mg of Suppocire AM (GATTEFOSSE) were dissolved in a mixed solution consisting of 2 mL of methylene chloride and 0.5 mL of ethanol. The thus obtained solution was emulsified into 5 mL of 0.5% polyvinyl alcohol (KURARAY CO., LTD., KURARAY POVAL 220C) - 0.1 M hydrochloric acid solution, at 8000 rpm for 3 minutes, using a homogenizer (IKA, Ultratalax T18). After completion of the emulsification, 20 mL of purified water was added to the reaction mixture, and the thus obtained mixture was then stirred using a stirrer for 3 hours, so that the solvent was distilled away. Aggregates were removed using a wire mesh sieve having a sieve mesh size of 149 µm, and fine particles were then recovered using a wire mesh sieve having a sieve mesh size of 20 µm. The fine particles were washed with purified water, and were then dispersed in a small amount of purified water, followed by freeze-drying for 1 day. The obtained fine particles were observed under a scanning electron microscope (Fig. 7).

### < Example 4 >

100 mg of Hypromellose phthalate (Shin-Etsu Chemical Co., Ltd., HP55), 200 mg of ethyl cellulose (Colorcon, ETHOCEL STD7), and 120 mg of Suppocire AM (GATTEFOSSE) were dissolved in a mixed solution consisting of 2 mL of methylene chloride and 0.5 mL of ethanol. The thus obtained solution was emulsified into 5 mL of 0.5% polyvinyl alcohol (KURARAY CO., LTD., KURARAY POVAL 220C) - 0.1 M hydrochloric acid solution, at 8000 rpm for 3 minutes, using a homogenizer (IKA, Ultratalax T18). After completion of the emulsification, 20 mL of purified water was added to the reaction mixture, and the thus obtained mixture was then stirred using a stirrer for 3 hours, so that the solvent was distilled away. Aggregates were removed using a wire mesh sieve having a sieve mesh size of 149 µm, and fine particles were then recovered using a wire mesh sieve having a sieve mesh size of 20 µm. The fine particles were washed with purified water, and were then dispersed in a small amount of purified water, followed by freeze-drying for 1 day. The obtained fine particles were observed under a scanning electron microscope (Fig. 8).

### < Example 5 >

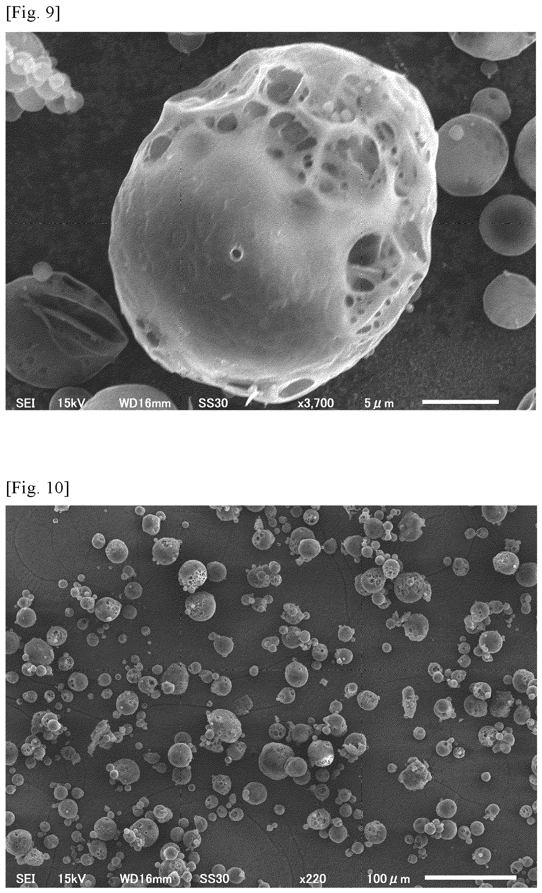

60 mg of Hypromellose phthalate (Shin-Etsu Chemical Co., Ltd., HP55) and 200 mg of ethyl cellulose (Colorcon, ETHOCEL STD7) were dissolved in a mixed solution consisting of 2 mL of methylene chloride and 0.5 mL of ethanol. The thus obtained solution was emulsified into 5 mL of 0.5% polyvinyl alcohol (KURARAY CO., LTD., KURARAY POVAL 220C) - 0.1 M hydrochloric acid solution, at 8000 rpm for 3 minutes, using a homogenizer (IKA, Ultratalax T18). After completion of the emulsification, 20 mL of purified water was added to the reaction mixture, and the thus obtained mixture was then stirred using a stirrer for 3 hours, so that the solvent was distilled away. Aggregates were removed using a wire mesh sieve having a sieve mesh size of 149 µm, and fine particles were then recovered using a wire mesh sieve having a sieve mesh size of 20 µm. The fine particles were washed with purified water, and were then dispersed in a small amount of purified water, followed by freeze-drying for 1 day. The obtained fine particles were observed under a scanning electron microscope (Fig. 9).

### < Example 6 >

60 mg of Hypromellose phthalate (Shin-Etsu Chemical Co., Ltd., HP55), 200 mg of ethyl cellulose (Colorcon, ETHOCEL STD7), and 120 mg of GELUCIRE 43/01 were dissolved in a mixed solution consisting of 2 mL of methylene chloride and 0.5 mL of ethanol. Into the thus obtained solution, 50 µL of a 0.5% Carbopol (BF Goodrich, Carbopol 934) aqueous solution and 5 µL of sorbitan monooleate were added, and the mixed solution was then subjected to sonification at 6 graduations of ChIP sonication under cooling on ice for 1 minute. The thus obtained solution was emulsified into 5 mL of 0.5% polyvinyl alcohol (KURARAY CO., LTD., KURARAY POVAL 220C) - 0.1 M hydrochloric acid solution, at 8000 rpm for 3 minutes, using a homogenizer (IKA, Ultratalax T18). After completion of the emulsification, 20 mL of purified water was added to the reaction mixture, and the thus obtained mixture was then stirred using a stirrer for 3 hours, so that the solvent was distilled away. Aggregates were removed using a wire mesh sieve having a sieve mesh size of 149 µm, and fine particles were then recovered using a wire mesh sieve having a sieve mesh size of 20 µm. The fine particles were washed with purified water, and were then dispersed in a small amount of purified water, followed by freeze-drying for 1 day. The obtained fine particles were observed under a scanning electron microscope (Fig. 10).

### < Example 7 >

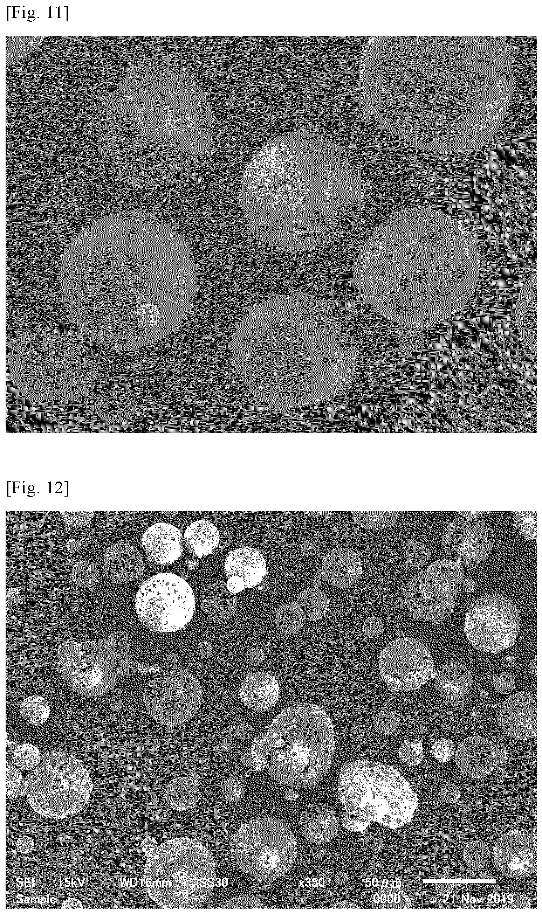

120 mg of Hypromellose phthalate (Shin-Etsu Chemical Co., Ltd., HP55), 400 mg of ethyl cellulose (Colorcon, ETHOCEL STD7), and 240 mg of GELUCIRE 43/01 (GATTEFOSSE) were dissolved in a mixed solution consisting of 4 mL of methylene chloride and 1 mL of ethanol. The thus obtained solution was emulsified into 10 mL of 0.5% polyvinyl alcohol (KURARAY CO., LTD., KURARAY POVAL 220C) - 0.1 M hydrochloric acid solution, at 6000 rpm for 3 minutes, using a homogenizer (IKA, Ultratalax T18). After completion of the emulsification, 40 mL of purified water was added to the reaction mixture, and the thus obtained mixture was then stirred using a stirrer for 3 hours, so that the solvent was distilled away. Aggregates were removed using a wire mesh sieve having a sieve mesh size of 149 µm, and fine particles were then recovered using a wire mesh sieve having a sieve mesh size of 20 µm. The fine particles were washed with purified water, and were then dispersed in a small amount of purified water, followed by freeze-drying for 1 day. The obtained fine particles were observed under a scanning electron microscope (Fig. 11).

### < Example 8 >

120 mg of Hydroxymethyl cellulose acetate succinate (DUPONT ANFINISOL AS 126G), 400 mg of ethyl cellulose (Colorcon, ETHOCEL STD7), and 240 mg of GELUCIRE 43/01 (GATTEFOSSE) were dissolved in a mixed solution consisting of 4 mL of methylene chloride and 1 mL of ethanol. The thus obtained solution was emulsified into 10 mL of 0.5% polyvinyl alcohol (KURARAY CO., LTD., KURARAY POVAL 220C) - 0.1 M hydrochloric acid solution, at 6000 rpm for 3 minutes, using a homogenizer (IKA, Ultratalax T18). After completion of the emulsification, 40 mL of purified water was added to the reaction mixture, and the thus obtained mixture was then stirred using a stirrer for 3 hours, so that the solvent was distilled away. Aggregates were removed using a wire mesh sieve having a sieve mesh size of 149 µm, and fine particles were then recovered using a wire mesh sieve having a sieve mesh size of 20 µm. The fine particles were washed with purified water, and were then dispersed in a small amount of purified water, followed by freeze-drying for 1 day. The obtained fine particles were observed under a scanning electron microscope (Fig. 12).

### < Example 9 >

### (Preparation of semi-porous Janus administration solution)

The fine particles (75 mg) of Example 7 were impregnated with 112.5 mg of a suspension, which contained, in 15 mg thereof, 10 U of human insulin (Nacalai Tesque, Inc., 28.5 U/mg), 1 mg of Labrasol (Gattefosse), 1 mg of decanoic acid, and 1 mg of polyoxyethylene hydrogenated castor oil (HCO60, Nikko Chemicals Co., Ltd.), and the resulting fine particles were then dried under reduced pressure at room temperature for 1 day. Thereafter, 90 mg of a 0.5% Carbopol (BF Goodrich, Carbopol 934) solution was added to the fine particle that had been dried under reduced pressure, so that the fine particles were impregnated with the Carbopol solution. After that, a water content was completely removed by drying under reduced pressure. Thereafter, 112.5 mg of Suppocire AM (Gattefosse) and 451.5 mg of soybean oil were added to the resultant, and the resulting fine particles were then dispersed using a bath sonicator to prepare a porous Janus administration solution. When 75 mg of this solution is administered to a rat with a weight of 170 g, it turns over that the rat contains 10 mg of the fine particles, 10 U of insulin, 1 mg of Labrasol, 1 mg of decanoic acid, 1 mg of polyoxyethylene hydrogenated castor oil, and 60 µg of Carbopol, per kg of body weight.

### (Control 1: Preparation of ethyl cellulose porous microsphere administration solution)

The fine particles (75 mg) of Comparative Example 1 were impregnated with 112.5 mg of a suspension, which contained, in 15 mg thereof, 10 U of human insulin (Nacalai Tesque, Inc., 28.5 U/mg), 1 mg of Labrasol (Gattefosse), 1 mg of decanoic acid, and 1 mg of polyoxyethylene hydrogenated castor oil (Nikko Chemicals Co., Ltd., HCO60), and the resulting fine particles were then dried under reduced pressure at room temperature for 1 day. Thereafter, 90 mg of a 0.5% Carbopol (BF Goodrich, Carbopol 934) solution was added to the fine particle that had been dried under reduced pressure, so that the fine particles were impregnated with the Carbopol solution. After that, a water content was completely removed by drying under reduced pressure. Thereafter, 112.5 mg of Suppocire AM (Gattefosse) and 451.5 mg of soybean oil were added to the resultant, and the resulting fine particles were then dispersed using a bath sonicator to prepare a porous microsphere administration solution. When 75 mg of this solution is administered to a rat with a weight of 170 g, it turns over that the rat contains 10 mg of the fine particles, 10 U of insulin, 1 mg of Labrasol, 1 mg of decanoic acid, 1 mg of polyoxyethylene hydrogenated castor oil, and 60 µg of Carbopol, per kg of body weight.

### (Control 2: Preparation of absorption enhancer-containing aqueous solution)

An aqueous solution was prepared, such that when 50 mg of the prepared solution was administered to a rat with a weight of 170 g, the rat contained 10 mg of the fine particles, 10 U of insulin, 1 mg of Labrasol, 1 mg of decanoic acid, 1 mg of polyoxyethylene hydrogenated castor oil, and 60 µg of Carbopol, per kg of body weight. The prepared aqueous solution was used as an absorption enhancer-containing aqueous solution.

### (Control 3: Preparation of insulin normal saline)

A normal saline was prepared, such that when 50 mg of the prepared normal saline was administered to a rat with a weight of 170 g, the rat contained 10 U of insulin. The prepared normal saline was used as an insulin normal saline.

### (Experiment regarding administration into rat small intestine)

Male Wistar rats with a body weight of 170 g were subjected to fasting overnight. Thereafter, an abdominal operation was performed on the rats, and the jejunum portion was removed from each rat. Thereafter, to a single rat, 75 mg of the porous Janus administration solution, 75 mg of the porous microsphere administration solution, 50 mg of the absorption enhancer-containing aqueous solution, or 50 mg of the insulin normal saline was administered using a syringe. Before and after the administration, 100 µL of blood was collected from the cervical vein over time, and was then centrifuged at 4°C at 16000 rpm for 3 minutes, so as to collect plasma. The collected plasma was cryopreserved at -80°C until the measurement of a plasma glucose level.

### (Measurement of plasma glucose level)

Using Glucose Assay Wako Kit (FUJIFILM Wako Pure Chemical Corporation), the absorbance at 505 nm was measured.

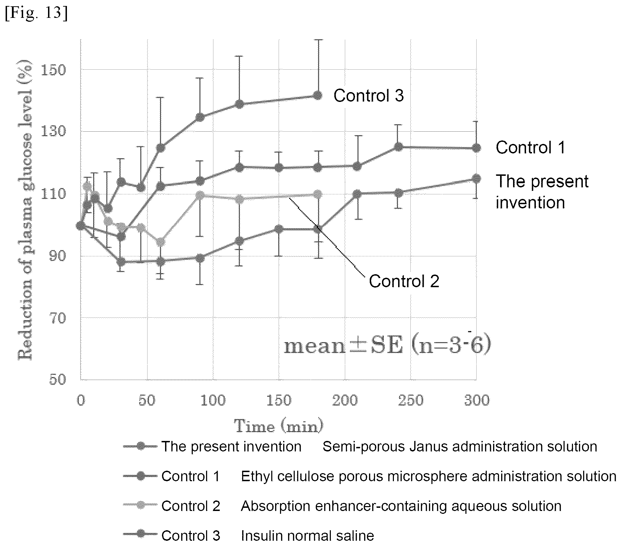

Transitions of plasma glucose levels after administration of individual preparations are shown in Fig. 13. In the case of the absorption enhancer-containing aqueous solution of Control 2 and the insulin normal saline of Control 3, the blood glucose level tended to increase due to tolerance to blood collection. In the case of the ethyl cellulose porous microsphere administration solution of Control 1, the increase in the blood glucose level as observed in Controls 2 and 3 was suppressed, but a decrease in the blood glucose level was only slightly observed. In contrast, in the case of the porous Janus of the present invention, hypoglycemic action was observed over 3 hours. Since the porous Janus of the present invention was used in impregnation with an aqueous suspension, it is considered that the absorption enhancer and the mucoadhesive polymer Carbopol are distributed to the hemisphere on the porous side. The porous side, on which Carbopol is distributed, is adhered to the mucosa, and insulin contained in the porous hemisphere is released onto the mucosa side.

At that time, it is considered that the non-porous hemisphere serves as a backing, and thus that drug release to the lumen side is suppressed. Moreover, it is also considered that the non-porous hemisphere is located on the lumen side and protects the porous hemisphere (containing insulin) that adheres to the mucosa side from enzymes and the like. In contrast, the porous microsphere does not have such a backing that it does not suppress the drug release to the lumen side or enzyme attacks. Therefore, it is considered that the porous Janus of the present invention could efficiently deliver the drug to the mucosa side of the absorption surface.

### < Example 10 >

A solution (HP55 solution), in which 390 mg of hypromellose phthalate (Shin-Etsu Chemical Co., Ltd., HP55) was dissolved in a mixed solution of 2 mL of methylene chloride and 0.5 mL of ethanol; a solution (EC-GE solution), in which 200 mg of ethyl cellulose (Colorcon, ETHOCEL STD7) and 120 mg of GELUCIRE 43/01 (GATTEFOSSE) 120 mg were dissolved in a mixed solution of 2 mL of methylene chloride and 0.5 mL of ethanol; a solution (EC solution), in which 320 mg of ethyl cellulose (Colorcon, ETHOCEL STD7) was dissolved in a mixed solution of 2 mL of methylene chloride and 0.5 mL of ethanol, were each prepared. Several droplets of each solution were spread on a glass-made Präparat, and were then dried by being left at room temperature overnight, thereby forming a film (i.e. an HP55 film formed from the HP55 solution; an EC-GE film formed from the EC-GE solution; and an EC film formed from the EC solution, respectively). Thereafter, 5 µL of purified water was placed on each film, and the contact angle was then measured according to a dropping method. The numerical value was calculated according to a θ/2 method. The results are shown in Table 1. Since the contact angle indicates wettability, the wettability became in the order of HP55 > EC > > EC-GE. In the fine particles of Example 7, the HP55 film corresponds to the composition of the porous hemisphere, whereas the EC-GE film corresponds to the hemisphere on which no pores are found. From these findings, it is considered that the contact angles among the hemispheres of the fine particles of Example 7 are largely different, and that if water is added to the present Janus fine particles, the porous hemispheres thereof having a small contact angle and good wettability are impregnated with the water. In addition, the wettability of the EC film was only slightly inferior to the wettability of the HP55 film. Thus, it is considered that a difference in the wettability among the hemispheres can be increased by addition of GELUCIRE 43/01, that distribution of a drug-containing aqueous solution onto the surface of a non-porous hemisphere with poor wettability can be avoided, and that a porous hemisphere with good wettability can be impregnated with the drug-containing aqueous solution.

**[Table 1]**

| Film | Contact angle |
|---|---|
| HP55 film | 53.7° |
| EC-GE film | 96.9° |
| EC film | 60.3° |

## Claims

1. A Janus fine particle having a particle diameter of 0.01 to 5000 µm, which is composed of a porous hemisphere portion and a non-porous hemisphere portion, wherein the wettability of the porous hemisphere portion to water is better than the wettability of the non-porous hemisphere portion to water.

2. The Janus fine particle according to claim 1, wherein at least the non-porous hemisphere portion of the Janus fine particle further comprises a wax.

3. The Janus fine particle according to claim 1 or 2, wherein the porous hemisphere portion at least comprises a polymer that swells due to water absorption.

4. The Janus fine particle according to any one of claims 1 to 3, wherein the porous hemisphere portion at least comprises an enteric polymer.

5. The Janus fine particle according to any one of claims 1 to 4, wherein the porous hemisphere portion at least comprises hypromellose phthalate or hydroxymethyl cellulose acetate succinate.

6. The Janus fine particle according to any one of claims 1 to 5, wherein the porous hemisphere portion comprises a drug.

7. The Janus fine particle according to claim 6, wherein the drug is a peptide, a protein, a nucleic acid, or a nucleic acid derivative.

8. The Janus fine particle according to any one of claims 1 to 5, wherein the porous hemisphere portion comprises an absorption enhancer, a protease inhibitor, a mucoadhesive polymer, or a sol-gel agent.

9. A method for producing the Janus fine particle any one of claims 1 to 7, comprising the following steps (i) and (ii):
(i) a step of emulsifying a solution prepared by dissolving one or more types of substances with good wettability and one or more types of substances with poor wettability in a common solvent, into a surfactant-containing aqueous solution; and
(ii) a step of drying the obtained emulsion to remove the solvent.

10. The method according to claim 8, wherein the volume of the surfactant-containing aqueous solution is 0.5 to 10 times higher than the volume of the solution prepared by dissolving one or more types of substances with good wettability and one or more types of substances with poor wettability in a common solvent.

11. The method according to claim 8 or 9, wherein, in the step (ii), the emulsion is dried for 30 minutes or more to remove the solvent.

12. The method according to any one of claims 8 to 10, which further comprises, after the step (ii),
(iii) a step of impregnating the porous hemisphere portion with an aqueous solution or suspension of a drug.

13. The method according to any one of claims 8 to 11, wherein the surfactant is a nonionic surfactant.

14. The method according to any one of claims 8 to 12, wherein the surfactant is polyvinyl alcohol.
